# EUROPEAN PATENT SPECIFICATION

(11) **EP 1 545 659 B1**
(45) Date of publication and mention of the grant of the patent: **09.07.2008**
(21) Application number: 03733835.7
(22) Date of filing: 03.03.2003
(51) Int. Cl.: A61M 5/168, A61M 5/172, G01J 3/02

(54) **DEVICE AND METHOD FOR QUALITATIVE AND QUANTITATIVE DETERMINATION OF INTRAVENOUS FLUID COMPONENTS**
VORRICHTUNG UND VERFAHREN ZUR QUALITATIVEN UND QUANTITATIVEN BESTIMMUNG VON INTRAVENÖSEN FLÜSSIGKEITSKOMPONENTEN
DISPOSITIF ET PROCEDE PERMETTANT LA DETERMINATION QUALITATIVE ET QUANTITATIVE DE CONSTITUANTS DE FLUIDE INTRAVEINEUX

(30) Priority: 04.10.2002 US 264666
(43) Date of publication of application: 29.06.2005
(73) Proprietor: Allgeyer, Dean Owen, Los Angeles, CA 90024 (US)
(72) Inventor: Allgeyer, Dean Owen, Los Angeles, CA 90024 (US)
(74) Representative: Müller, Frithjof E.
(86) International application number: PCT/US2003/006162
(87) International publication number: WO 2004/033003

(56) References cited:
- WO-A-98/19592
- WO-A-98/56028
- WO-A-99/62574
- US-A- 6 111 639
- US-B1- 6 236 041
- US-B1- 6 261 262

## Description

### FIELD OF THE INVENTION

This invention relates generally to devices and methods of preventing medication errors. Specifically, utilization of spectroscopy for the qualitative and quantitative determination of fluid path components is disclosed. Spectroscopy can be applied to intravenous infusions where the determination of fluid path components can significantly decrease and prevent the occurrence of medication errors.

### BACKGROUND OF THE INVENTION

Medical errors are recognized as a serious problem associated with the delivery of health care. The Institute of Medicine estimates that as many as 98,000 Americans die annually as a result of preventable medical errors. Medication errors constitute a critical component of medical errors in general. The Joint Commission on Accreditation of Healthcare Organizations (JCAHO) stated in the November 19, 1999 Sentinel Event Alert, "Medication errors are one of the most common causes of avoidable harm to patients in health care organizations." The 2001 publication "Retrospective Analysis of Mortalities Associated With Medication Errors," Am. J. Health-Syst. Pharm., Vol. 5 8, Oct 1, 2001, reported that among fatal medication errors, improper dose (40.9%) and administering the wrong drug (16%) were the most frequent. The FDA 2002 Performance Plan proposes the development of standards to prevent dosing and drug mix-ups.

Medication errors are attributable to one or more of fourteen causes that span the range from conceiving a therapeutic regimen to the delivery of a pharmaceutical compound. The top six mistakes, according to the *Am.J. Heath-Syst. Pharm.* article, constitute 80 per cent of all medication errors and are, in decreasing frequency, wrong dose, wrong drug, wrong route of administration, wrong patient, wrong rate, and wrong concentration. A number of commercially available products attempt to address the need to decrease the incidence of medication errors and are directed at various aspects of the possible mistakes. These products utilize software, drug libraries, and institutional limits placed on specific drugs.

For example, Alaris Corporation markets a software system named Guardrails^{™}. Guardrails^{™} provides protection against infusion programming errors with tests of reasonableness during the initial programming and in subsequent programming of intravenous (IV) infusions. The system includes pre-programmed drug maximums that cannot be overridden by faulty bedside data input. The system, however, still leaves the potential for wrong drug, wrong patient, wrong rate, and wrong concentration errors.

Picis Corporation also offers medication management and data collection software trademarked as CareSuite^{™}. An excerpt from its literature states: "Simply obtaining the infusion dose or rate from the intravenous pump would not be enough without automatically identifying the drug being administered. Thus the goal was to minimize computation and recording errors and give clinicians greater control over fluid management. With automatic data collection by CareSuite^{™} directly from the infusion pump, clinicians can be assured of accurate fluid infusions. Medical errors related to incorrect data entry and incorrect calculations are reduced."

Systems like Guardrails^{™} and CareSuite^{™}, while helpful in decreasing medication errors, all suffer from the same vulnerability, namely operator error. One such error arises, for example, when the wrong drug concentration, or even the wrong drug, is mixed by a hospital pharmacy. As the intravenous administration systems cannot identify the composition or concentration of an intravenous infusion, drug swap or admixture concentration errors are not prevented. U.S. Patent No. 6,070,761 to Bloom et al attempts to deal with this problem during the vial loading process by standardizing and automating drug mixture and then verifying that the drug is the correct one for a given patient. Networking to one or more databases performs the verification. The system envisions various security and quality control features such as bar codes and passwords. Although this system may potentially lessen the likelihood of human errors, it cannot eliminate them, because it cannot positively identify the infusate.

Another unfortunate issue has been that of medical personnel deliberately administering unprescribed, dangerous, and even fatal types or doses of medication. One reported case resulted in the criminal conviction of a medical professional who administered muscle relaxants to ICU patients, resulting in a number of deaths. Currently, there is no system to automatically alert medical staff to this kind of danger. Errors of omission also occur in the administering of medications. As an example, such an error could occur when scheduled doses, whose timing is critical, are omitted or administered at unscheduled times. A system that automatically identifies the drug, time of administration, and dosing would serve as a quality control system to prevent inadvertent medication errors, as well as a deterrent to the deliberate misuse of the drugs or the omission of scheduled doses.

The pharmaceutical manufacturing industry, where real-time, on-line analysis of compounds is helpful for quality assurance and compliance with FDA GMP (Good Manufacturing Procedures) regulations, has automated systems that perform qualitative and quantitative analysis. These systems provide an automated analytical capability using spectroscopic analysis. A spectrometer passes a continuous portion of the electromagnetic spectrum through a specimen to develop an absorption spectrum. To establish a compound's identity, the absorption spectra can then be compared to spectral libraries of specific compounds. This identification process can be digitally automated. Quantitative analysis based on chemometric algorithms is then possible. The Beer-Lambert law, where all variables except concentration are known, can be used to perform the quantitative analysis. In present commercial applications, spectral databases use wavelengths in the range of 1-15 microns. The commercial devices, however are too large and too expensive for incorporation into clinical use.

Medical applications of spectroscopy are known. For example, Robinson, in U.S. Patent No. 6,278,889, discloses a quantitative spectroscopic system to noninvasely determine *in vivo* glucose concentrations. Robinson also discloses the basics of quantitative spectroscopy. Unfortunately, Robinson's system is complex and costly due to 1) a variable path length and 2) a multiplicity of absorbing substances within the light path other than the analyte of interest.

U.S. Patent No. 6,122,536 to Sun et al discloses an *in vivo* invasive infrared (IR) emitter and sensor for quantitative determinations of circulating analytes. The complexity of this system is due to the multiplicity of analytes and tissue components lying within a variable and, at times, a changing optical path length. The ideal situation for qualitative and quantitative spectroscopy exists when a single compound is suspended evenly in a relatively non-absorbing and spectrally known medium, and enclosed in a vessel relatively transparent to electromagnetic radiation and having a known spectral profile. Ideally, the vessel has a fixed, known optical path length. A fixed and known path length would, however, be unnecessary in systems utilizing Raman spectroscopy. Reflectance-based systems and qualitative systems without quantitative capability would also not require a fixed optical path length.

Rapid advancements in the areas of solid-state electronics, optoelectronics, and microprocessors have allowed the commercial production of high quality and inexpensive components in spectroscopic analysis. Various combinations of hardware and software can now perform rapid spectral data acquisition and analysis functions inexpensively. Medical and non-medical applications of these technologies are known. For example, pulse oximetry utilizes an LED-photodiode arrangement whereby two frequencies are sequentially passed through perfused tissue to determine oxygen saturation levels of hemoglobin. The transmittance ratios of these two wavelengths are then compared to an empirically derived and stored nomogram to determine the percentage of oxygen saturation. One example of such a device is U.S. Patent No. 4,621,643 to New, Jr. et al.

The present wavelength range of commercial LED's is about 400-1,600 nanometers, or 0.4-1.6 microns. One example of an LED application, U.S. Patent No. 5,995,858 to Kinast, discloses a phase-shifted, dual LED oximeter, which increases the signal to noise strength. The LED-photodiode probes used in the oximeter are the size of a band-aid and are inexpensive enough for a single, disposable use. Rosenthal, in U.S. Patent No. 4,286,327, has devised an LED-based system for quantitative analysis of grain. McDonald, in U.S. Patent No. 6,072,576, discloses an on-line, real-time Fourier Transformed near infrared (FTNIR) based system for process control in a chemical plant. Similarly, Ditmarsen, in U.S. Patent No. 6,236,048, discloses a spectrally based system for characterization of a flowable material. Axon, in U.S. Patent No. 6,362,891 discloses an FTNIR system for quality control of pharmaceutical tablet ingredients. It is noted that permissible tolerance levels of the ingredients are set for comparison to known levels, but thereafter unskilled operators are able to operate the system. Schnell, in U.S. Patent No. 4,620,284, discloses a Raman-based system for qualitative and quantitative analysis using primarily a photodiode array for signal collection prior to comparison with known spectra.

To provide safer intravenous infusion would require that any qualitative or quantitative analysis of the infusate have some direct operative connection with the infusion process. Medications are often administered intravenously in a hospital setting utilizing a bag containing the added medication, an intravenous administration set, and an infusion pump. One conventional type of infusion pump system employs a peristaltic pump in conjunction with an intravenous administration set. The administration set consists of flexible thermoplastic tubing through which fluid flows from a suspended container, such as a flexible bag or rigid bottle, to a patient's vein. Much of the prior art relating to pumps is directed to delivering an accurate infusion rate, because an inaccurate infusion rate will lead to potentially dangerous incorrect doses of medications and fluids. One example of an infusion pump is U.S. Patent No. 6,261,262 to Briggs et al. IV administration sets, such as U.S. Patent No. 5,226,886 to Skakoon et al, provide tubing that carries the intravenous fluid through the infusion pump and to the patient.

Numerous models of IV infusion pumps and IV administration sets are commercially available. A typical system would be like that of the Horizon family of pumps and Horizon IV sets sold by B. Braun. All pumps in this family deliver infusions by positive pressure, which is generated through a volumetric displacement reservoir. This reservoir is incorporated within the IV administration set tubing, which is loaded into the pump via a door mechanism. The pump is set to run at a certain rate and activated. Alarms typically indicate some or all of the following problems: air-in-line, container empty, door open, downstream occlusion, hold time exceeded, low battery, low flow, system error, and upstream occlusion. Other common features include a dose/rate calculator mode, which automatically calculates the rate when dose information is entered, or the dose when rate information is entered. "Smart Pumps" can have preprogrammed institutional drug limits. The newest pump in the Horizon series, the Outlook ^{™} incorporates bar code scanning technology to reduce drug administration errors. Even this technology, however, cannot account for errors such as incorrect admixture or incorrect labeling of the drug containers.

Ultimately, the existing safety systems related to intravenous infusion attempt to ensure that the right drug, in the right dose, is given to the right patient, with the right route of administration, at the right rate and strength, and at the right time. They all fail, however, because they lack a real-time ability to identify pharmaceutical or other compounds while these substances are being delivered to the patient. Such a system would further reduce medication errors. It would also permit data capture, storage, and analysis. Such a system could, for example, be integrated with a computer order entry system to form a complete feedback loop for quality assurance, maintenance of patient records, generation of bills, or even research and statistical analysis. It could, for example, eliminate the need for nurses to chart the administration of intravenous medications. At the present time, no mechanism exists that can perform these functions, because no automated mechanism exists for the qualitative and quantitative analysis of the drugs as they are intravenously provided to a patient.

US Patent 6,111,639 discloses the addition of a predetermined amount of a coloring material such as a vegetable dye to a container such as an intravenous bag with the coloring material defining a specific type and concentration of a medication. The coloring material is analyzed to determine the medication present and the concentration.

It is an object of the present invention to provide an infusion pump system for delivering an intended intravenous fluid containing at least one medication to a patient, which infusion pump system provides a real time ability to identify pharmaceutical or other compounds while these substances are being delivered to the patient to further reduce medication errors.

A further object is to provide an improved intravenous administration set which can be used for said infusion pump system.

The present invention solves these objects in accordance with the enclosed claims.

### SUMMARY OF THE INVENTION

The safety and efficiency of the administration of intravenous pharmaceuticals can be improved by adapting the infusion process for spectroscopic analysis. This approach is feasible and would solve many of the problems previously identified. The medications are individually mixed with carrier solutions that are weak absorbers in the ultraviolet (UV), visible (VIS), and near infrared (NIR) range. These solutions are administered with intravenous administration sets made from polyvinylchloride or other similar polymers, which are also weak absorbers in the UV-VIS-NIR region. Typical medication carriers, such as 5% dextrose in water or 0.9% sodium chloride solution, and the polymers within the intravenous (IV) administration sets, have known spectral profiles which can easily be compensated for in the qualitative and quantitative determinations of the components of interest.

The present invention qualitatively and quantitatively determines fluid path components in intravenous infusions for medical patients. In one preferred embodiment, it utilizes an IV administration set containing an optical window in conjunction with an IV infusion pump. Preferably, the pump contains embedded hardware and software to perform the spectral analysis of the fluid path components. By implementation of the present invention, errors of medication administration may be prevented. In addition, the invention may include warning mechanisms, fail-safe infusion shut-offs, means for automated recording of administered drugs, and other features facilitating administrative documentation and quality control.

In one embodiment of the invention, an infusion pump is used in the spectroscopic analysis of an intravenously delivered fluid. The analysis identifies one or more of the fluid's components, and preferably also determines their concentration. The pump includes a housing with an exterior and an interior. An intravenous administration set is used to deliver the fluid. The fluid follows a path through the interior of the pump housing. An emission source disposed within the housing directs a predetermined spectrum of electromagnetic radiation (EMR) at the fluid. A detector disposed within the housing detects that portion of the radiation transmitted, absorbed or reflected by the fluid, depending on the type of spectroscopic system. The detector produces an electronic signal used to spectroscopically analyze a component or components of the fluid. In the preferred embodiment of the invention, light-emitting diodes (LED's) are used for the emission source, and the exterior of the pump has controls and a display for controlling the spectroscopic analysis. In other embodiments of the invention, the control of the analysis and the display of its results can be performed remotely by various peripheral devices like keyboards and monitors.

In another embodiment, the invention comprises an intravenous administration set having tubing to supply intravenous fluid to a patient. The administration set includes an optical window that permits the transmission and detection of EMR for use in a spectroscopic analysis of the fluid's components. In the preferred version of this embodiment, the optical window is in an optical chamber that creates a more precise optical path length for the spectroscopic analysis.

As a result of the present invention, the medical field can now prevent wrong drug and wrong concentration errors. This ability can now be combined with existing or easily adaptable electronic devices and software to also minimize or eliminate errors relating to wrong dose, wrong concentration, wrong administration time, and even wrong patient. Even in the simplest of embodiments, the present invention will provide improved care and increased deterrence. Nurses and other medical personnel will be more attentive knowing that any incorrect drug or improper dose will be recorded, and it will be more likely that system errors can be identified. Additional features and advantages of the present invention will become apparent when considered in conjunction with the accompanying drawings and the detailed description below.

### BRIEF DESCRIPTION OF THE DRAWINGS

Figure 1A is a perspective view of an intravenous administration set.
Figure 1B is a perspective view of an infusion pump that is mounted on a stand and can be used with the administration set.
Figure 2 depicts an optical chamber with recessed optical windows.
Figure 3 is a plan view along section B-B of Figure 2, depicting the recessed optical windows within the optical chamber.
Figure 4 schematically depicts a pole mounted pump housing with an LED array, photodetector, and an indentation to position the optical chamber.
Figure 5 is a block circuitry diagram of an LED array and photodetector schematically depicted in Fig. 4.
Figure 6 is a timing diagram for a spectroscopic analysis using three LED's and depicts sampling intervals and output concentrations.
Figure 7 shows the IR spectra of morphine, dilaudid, and demerol.
Figure 8 schematically depicts a pole-mounted IV pump housing with an infrared emission source and a photodiode array detector.
Figure 9 depicts a portion of an IV administration set with an optical window.
Figure 10 depicts an optical housing covering the optical window.
Figure 11 is a cross section of a two-way fiberoptic bundle.

### DETAILED DESCRIPTION OF THE PREFERRED EMBODIMENTS

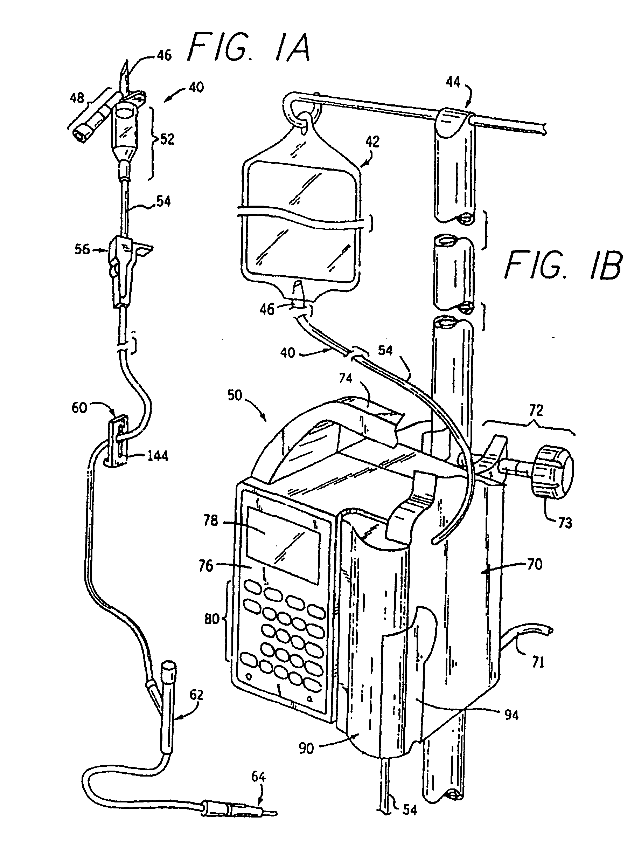

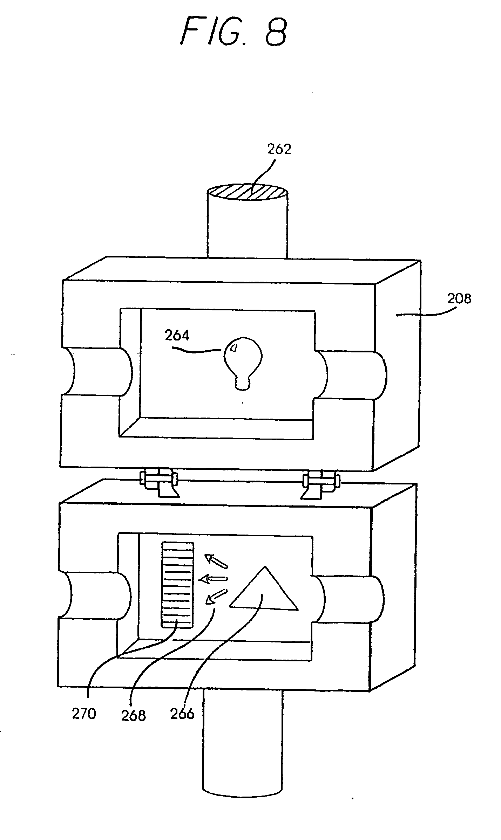

Two examples of the present invention are discussed below in connection with Figures 1-11. The first example uses a three LED array to check for the presence of morphine HCL. It is discussed with reference to Figures 1-7. Morphine is used as an example because the JCAHO has identified narcotics as one class of agents involved in a significant number of medication errors. The embodiment in Figures 1-7 might be used, for example, in Patient Controlled Analgesia (PCA) pumps. PCA pumps are dedicated for use typically with one of three narcotics: morphine, dilaudid, or demerol. An LED-based system for this application would be inexpensive and would be able to qualitatively and quantitatively function with a minimum of spectral data points. The second example, in Figures 8-11, utilizes a photodiode array collector and IR broadband emission source. This arrangement could be employed for a system that processes a large number of different pharmaceutical compounds. It could be updated by the inputting or downloading of reference spectral data to its memory.

FIG. 1A depicts a typical IV administration set 40. Figure 1B depicts a typical peristaltic infusion pump 50 that measures the flow rate of intravenous fluids being delivered through administration set 40. The administration set 40 is typically employed to deliver parenteral fluids, medications, whole blood, red blood cell components, and the like from a fluid container, such as a bottle or flexible bag 42. Bag 42 is shown in FIG. 1B supported on an intravenous administration stand 44. A portion of the administration set 40 is engaged by the peristaltic pump 50, and a distal portion of the administration set 40 downstream of the pump 50 can be connected to a patient's indwelling vein access device, such as a needle or cannula (not illustrated), which is inserted into the patient.

The administration set 40 has a hollow piercing pin 46 projecting from a conventional bacterial filter 48 at the upper end of a drip chamber 52. A length of hollow flexible tubing 54 extends from the bottom of drip chamber 52 through a roller clamp 56. Disposed on tubing 54 downstream of the roller clamp 56 is a slide clamp 60. A conventional Y-injection site 62 is provided on the tubing 54 downstream of the slide clamp 60. The distal end of the tubing 54 is provided with a conventional male adaptor 64, which is designed to be attached to a venipuncture device (not shown). The IV administration set in Fig. 1A and adapted for use with the present invention is preferably constructed of a standard medical grade polymer such as polyvinylchloride (PVC), which has known and minimal absorption in the UV-VIS-NIR wavelengths.

Other typical IV administration sets are configured differently and may have more or fewer components. For the purpose of the present invention, the important aspect of the administration set is the tubing that carries the fluid through the infusion pump and to the patient. Thus, the use of the term IV administration set can also be understood broadly to be just the tubing. To the extent the present invention may require other features to operate, the administration set should be understood to include typical features of an administration set, such as drip chamber 52, roller clamp 56, adaptor 64, or a venipuncture device.

As shown in FIG. 1B, the pump 50 includes a housing 70 and a clamp 72 with knob 73 by which the pump 50 can be mounted to stand 44. A handle 74 projects upward from the top of the housing 70. The exterior of pump 50 has a front panel 76 containing a liquid crystal display 78 and a key pad 80. Next to front panel 76 is a front door 90 with handle 94. Tubing 54 passes into, through, and out of the interior of pump 50, where the flow rate of the intravenous fluid is physically controlled according to parameters entered on key pad 80.

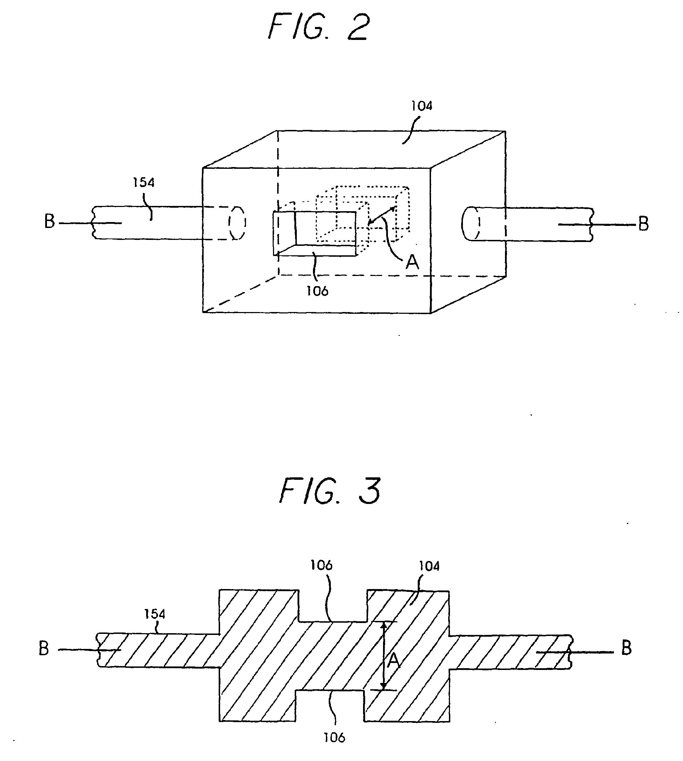

Figure 2 shows tubing 154 passing through optical chamber 104. Preferably optical chamber 104 consists of a fixed dimension, parallel-walled structure configured to be included in an infusion pump housing. Optical windows 106 are physically recessed within optical chamber 104. This protects the optical windows 106 from damage or smudging, which in turn could distort EMR absorption and reflection values. IV tubing 154 and optical chamber 104 with fixed optical path length A would be primed with the infusate. A fixed length optical path would not be a requirement of a solely qualitative, transmission-based system or a system based on reflectance or Raman spectroscopy.

Figure 3 is a plan view through section B-B of Figure 2 showing recessed optical windows 106 delineating path length A. As depicted in Fig. 3, the optical window106 is that part of the optical chamber through which EMR traverses. It may or may not be recessed, may or may not be of the same composition as the optical chamber, and may be positioned on both sides of the optical chamber in transmission-based systems. It could, however, in the case of a reflectance or Raman system be a single window on one side of chamber 104. As discussed below with respect to other possible embodiments, the term optical window should, where appropriate, be interpreted in its broadest sense as permitting the passage of EMR. Alternatively, the one depicted in Fig. 3, it can have a discrete physical structure, such as the one depicted in Fig. 3.

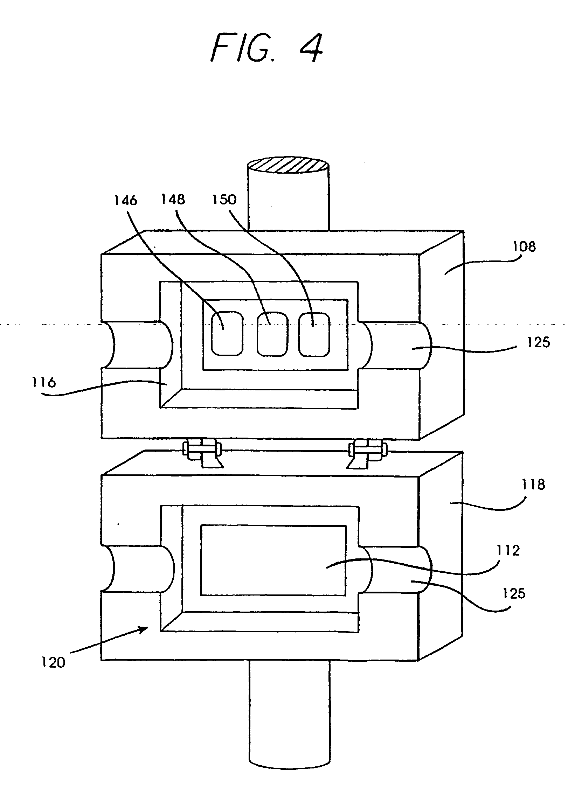

Figure 4 schematically depicts a hinged intravenous pump housing 108 in the open position. Only aspects of the present invention are shown and described. Other conventional aspects related to pump control, monitoring, warning and the like are not shown. An array of three LED's 146, 148, 150, and photodetector 112 are located in receptacle indentation 116, in the pump's interior 120. Notch 125 is adapted to hold tubing 154. Loading optical chamber 104 into infusion pump 108 and closing door 118 linearly aligns optical chamber 104 between LED array 110 and photodetector 112. Mirrors, choppers, fiberoptic bundles, and other optical devices (none of which are shown) can be utilized to deliver the EMR in various ways, such as a point source. In the preferred embodiment, tubing 154 includes optical chamber 104 that provides the fixed optical path required for quantitative analysis. It is also possible to eliminate optical chamber 104 and secure tubing 154 in a predetermined path in the pump's interior. Simply securing tubing 154 between LED's 146,148, 150 and photodetector 112 may provide an optical window through the tubing with enough of a fixed optical path length without varying the cross-sectional configuration of the entire length of tubing. There is concern, however, that the flexibility and roundness of tubing 154 may vary the optical path length from one use to the next, depending on how the tubing is manually placed in the pump. That is why the preferred embodiment includes optical chamber 104. In Figure 4, notch 125 and indentation 116 hold tubing 154 and optical chamber 104 securely within the pump interior 120. The tubing154 and optical chamber 104 provide a path for the intravenous fluid that takes it between LED array 110 and photodetector 112 to enable the spectroscopic analysis. The term fluid path should be understood to encompass a meaning necessary to understand the claims and to enable the present invention. The path, for example, may simply be a portion of tubing or it may be a location inside the pump where the tubing is placed.

In one embodiment of the invention schematically depicted in Figure 4, the exterior of pump housing 108 can include a key pad (not shown) and display (not shown) similar to key pad 80 and display 78 in Figure 1B. The key pad can be used to preprogram numeric, alphanumeric, or even alphabetical information that identifies important parameters such as the identity of the fluid components to be analyzed, the maximum allowable concentrations of medication, information related to dose scheduling, and even administrative options such as patient name or number and whether the information should be saved or transferred to a remote database. In the preferred embodiment the fluid components are medications, or even components of the medications, that can be used to identify medication being administered to a patient.

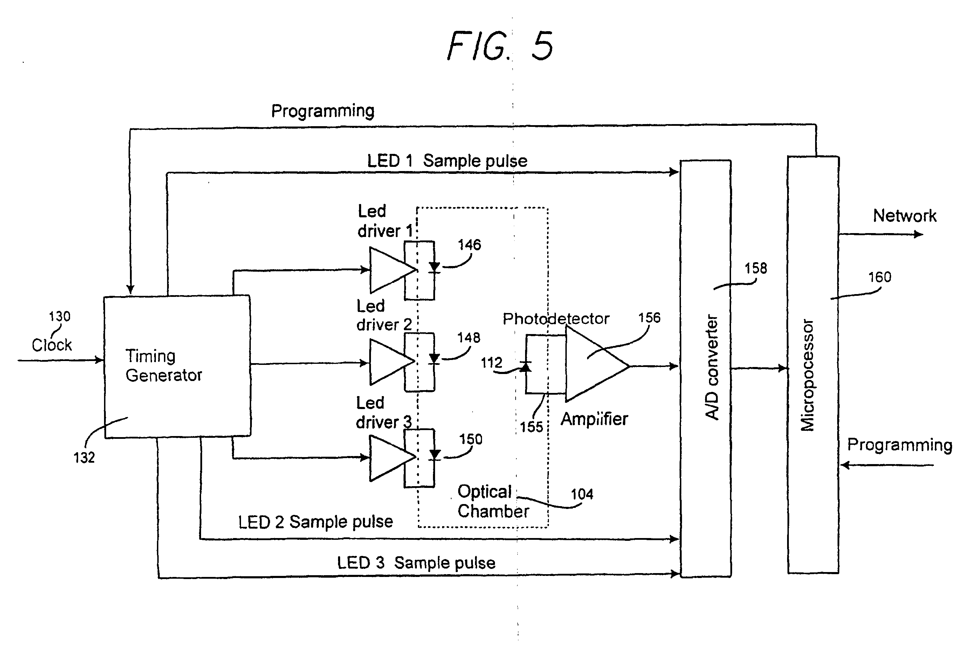

Figure 5 is a diagram of the spectroscopic analysis. Clock 130 controls timing generator 132, which sequentially drives LED's 146,148,150. The optical output traverses the optical windows 106 (not shown) and optical chamber 104 and the infusate containing the compound of interest. The optical output is detected by photodetector 112. Photodetector 112 sends signal 155 to amplifier 156. The amplifier output is sampled by the A/D converter 158 as allowed for by LED timing sampling pulses 1, 2, and 3. The final output signal is sent to pre-programmed or programmable microprocessor 160, which can also control LED signal generation. Microprocessor 160 is preferably programmable and can provide network interfacing capability. Present LED wavelengths range from approximately 400 to 1600 nanometers. It is understood that longer wavelengths are under development.

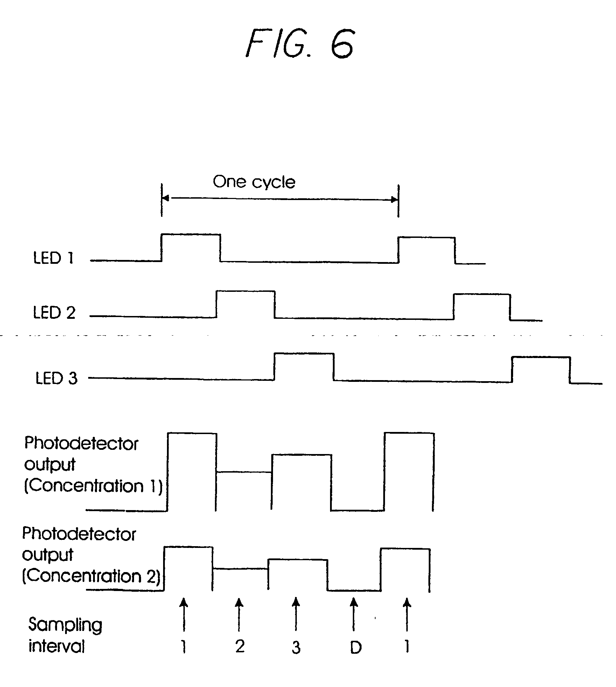

Figure 6 shows a timing diagram in which the three LED's are sequentially driven, with one dark sampling interval. The dark period allows the continuous subtraction of ambient stray light from the LED transmissive values. One cycle is shown, with typical drive frequencies on the order of 300Hz. The photodetector signal strength outputs are shown as a bar graph. This ratio of photodetector output is unique to and constitutes the unique identifier for any single pharmaceutical. The number of wavelengths necessary for the analysis may vary, depending on the number of pharmaceuticals in the universe and their underlying spectra. Though absolute signal strength varies, the ratio of signal strengths at the given wavelengths are concentration independent and constitute the unique characteristic of a particular compound, regardless of concentration. This ratio of signal strength is used to identify the compound by virtue of a comparison to known stored values. Photodetector outputs for single and double strengths of the same pharmaceutical compound are shown in Fig. 6 to illustrate this point: The output from the double strength solution of concentration 2 is Half that of concentration 1, but the transmittance ratios remain the same.

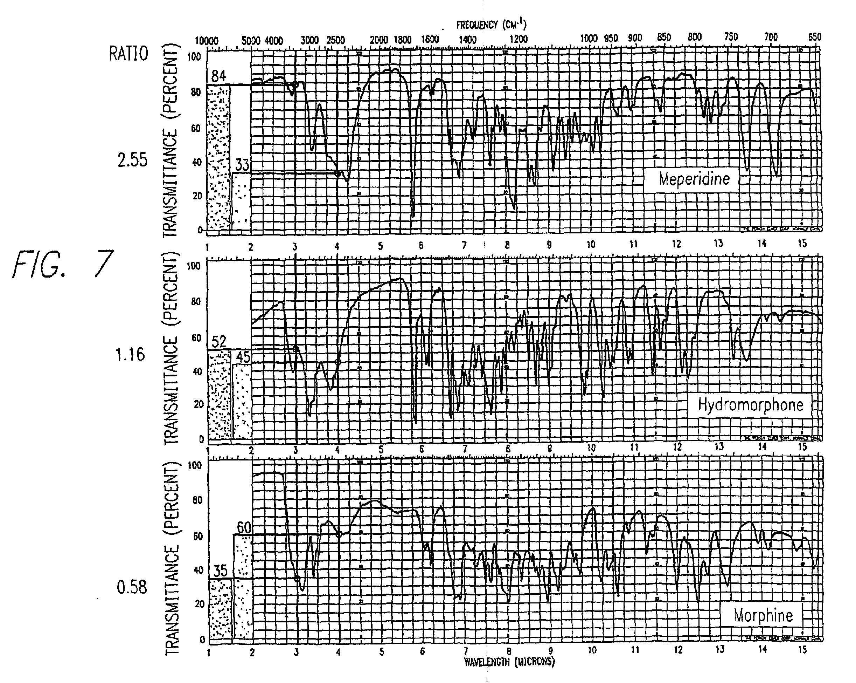

Figure 7 shows the IR spectrum of morphine, hydromorphone, and meperidine. One can see that as few as two wavelengths are needed to generate a unique identifying ratio that enables differentiation in this limited drug universe. For example, percent transmittance corresponding to the 3 and 4 micron wavelengths are shown at the left in a bar graph format. Dividing the percent transmission at 3 microns by that at 4 microns results in the unique identifying ratios in the left-hand margin. It is appreciated that these ratios are concentration independent. Hence in some instances, a two-wavelength photodetector arrangement could be utilized with minimal cost.

A Patient Controlled Analgesia (PCA) pump provides a basic example. A hospital wants a qualitative confirmation (yes or no) and quantitative information (dose or concentration) concerning a drug universe of morphine, dilaudid, and demerol, for which it utilizes a dedicated PCA pump. In the present example, the nurse may input the drug, concentration, and patient identifier information. The pump would have its own microprocessor-based control and data handling functions for rate control and spectroscopic analysis. A keypad and display panel on the outside of the pump could be used to perform data input. Alternatively, one could use a separate monitor and keyboard, as with personal computers. The control and analysis functions could be provided through a network to the pump, in a patient's room, a nurse's station, or elsewhere. The drug container could be bar coded and scanned by the pump for comparison to computer-entered orders for the patient. Once the pump is programmed, the infusion is begun.

The pump can be programmed at the factory or at the hospital, or portions of the programming could be performed at each location. The type of programming would be determined by the number of specific pharmaceuticals and quantitative and qualitative data required. The choice of various hardware and software aspects of the programming and control of the pump are left to the designer. The pump would have a number of LED's as the emission source, some or all of which would be activated based on the unique identifier characteristic of the narcotics in the hospital formulary. These LED's and their respective outputs would then be activated in a timed manner such that the photodetector output ratios are concentration independent and able to qualitatively differentiate the compounds.

As an example, a three LED system might have four discrete sampling intervals for calculation purposes. In the first interval, the first LED would be energized and the emission would pass through the sample and be detected by the photodetector. The next interval would contain the photodetector signal from the second LED, and the third interval would contain the photodetector signal from LED 3. Interval four would sample signal generation due to ambient light without LED activation, which would be subtracted from all LED signals prior to calculation of the relative transmissive values.

The transmission ratio generated by the three LED array would then be calculated and compared by algorithm to known transmission values. The identification could be performed as a YES or NO query. YES causes the software to proceed to the quantitative aspect of the algorithm, if desired. NO leads to one of several actions depending on the programming. Most likely the programming will cause the infusion to stop and will give a readout of the error involved.

The calculation of the transmissive ratios, which are concentration independent because they reflect the relative signal strength output at specific wavelengths, allows the system flexibility to deal with the absorptivity of varied concentrations. Concentrations will vary with dosage, admixture techniques, etc. Transmissive ratios will, however, remain constant regardless of concentration and will function as the unique identifier for any given compound.

The quantitative analysis begins by selecting at least one wavelength which has a known percent transmission greater than 0 and less than 100 percent. If all other variables except concentration are known, the Beer-Lambert equation can solve for concentration. At a fraction of 300Hz, the concentration is determined, multiplied by the infusion rate, and then integrated over time to tabulate the total dosage. For safety, it is preferable that the step of qualitative confirmation always be performed before any quantitative analysis. Otherwise, programming only for a quantitative analysis of the drug risks propagating a wrong drug error. The hardware and software components for using the spectroscopic data will vary according to the user's requirements. Different healthcare facilities have different clinical and administrative needs. Simpler requirements may include qualitative identification and/or quantitative data for a single agent. More sophisticated requirements could necessitate both qualitative and quantitative analysis for a complete and ever expanding pharmacopoeia.

In the situation where a hospital desires an expanded ability to qualitatively and/or quantitatively determine fluid path constituents, a system utilizing a photodiode array detector could be employed. This system would likely cost more than a basic LED system but would have the advantage of generating a vast number of spectral data points quickly and accurately. Many types of equipment could be employed, including different emission sources, such as a tungsten, halogen, or incandescent lamp for generating a broad spectrum of wavelengths. A variety of detectors could also be used, such as photovoltaic and photoconductive cells, vacuum phototubes, photomultipliers, photodiodes, and charge coupled devices. The examples of an LED and PDA-based system are given as preferred examples of an inexpensive system, but they are not intended to limit the scope of the invention. Various embodiments of the invention with any given emission source would function well with an appropriately matching detector.

Figure 8 depicts an alternative embodiment where the emission source is a broadband infrared emitter and the data generated is absorbance. The emission source could use various EMR wavelengths, depending on the underlying spectra of the pharmaceutical universe. Likewise, reflectance, Raman, or any other spectral data could be gathered and analyzed. IV pump housing 208 is mounted on pole 262 and is again schematically depicted without details relating to infusion rate components. Emission source 264 in this embodiment emits over the range of 1-15 microns, which is the typical range of existing commercial spectral databases. This range allows "fingerprinting" of compounds, i.e., the determination of fluid components independent of their concentration. This data is digitally compared to stored reference data at predetermined confidence levels. Many mathematical algorithms are capable of analyzing the data.

The emission from source 264 traverses the optical window and optical chamber containing the IV infusate (not shown) and is diffracted by prism 266 or a holographic grating (not shown). Diffracted radiation 268 is detected by photodiode array 270 and generates a multiplicity of signals. A CPU (not shown) controls the process, is programmable, and can acquire an entire spectrum in a fraction of a second. As before, the relative ratios acquired are the basis for a concentration independent identification of the compound when compared to stored spectral data. This process is repeated rapidly and the results averaged on an ongoing basis. Quantitative analysis can also be performed using chemometric analysis where the path length of the optical chamber is known and is integrated over time with the flow rate from the pump to result in a summation of dose delivered over time.

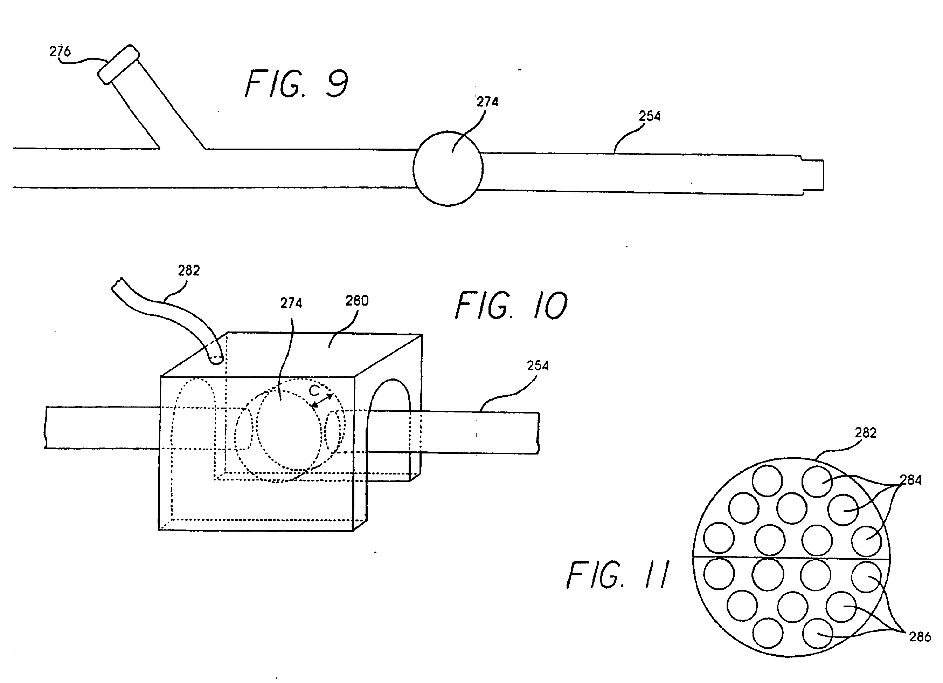

Figure 9 shows a portion of an intravenous administration set with tubing 254, an optical chamber 274, and infusion port 276. In Figure 10, optical housing 280 engages and covers optical chamber 274. Optical path length C is fixed between opposing walls of optical chamber 274. The walls of chamber 274 could be the optical windows through which the EMR passes. This arrangement would be used in situations such as the operating room, where IV medications are delivered by IV bolus. Here it would be advantageous to physically distance the emission source and detector hardware from the optical housing 280 by means of fiber optic cable 282. In Figure 11, fiberoptic cable 282 is shown in cross section. Fiberoptic bundles 284 transmit the emission electromagnetic radiation to housing 280, and fiberoptic bundles 286 transmit the collected electromagnetic radiation to the detector hardware and software.

The preceding discussion of the present invention covers its basic structure and process. Those of skill in the art will realize that changes can be made in the fabrication and configuration of the invention. For example, the description of the invention contemplates that an emission source and a detector will be permanently mounted in the pump housing, and that the spectroscopic analysis of the signals generated by the detector can be performed by the pump. This is accomplished by electronic hardware and/or software known or easily developed by those in the art. The information could be displayed by the pump, or it could be transmitted to and displayed at a remote location such as a nurse's station, or displayed at both locations. Similarly, the controls for the spectroscopic analysis could be pump mounted, remotely located, or both. The analysis can be performed by any combination of hardware and/or software. For example, pumps could use modules with different hardwired or programmable chips that could be swapped in and out of different pumps. Each chip would represent the analytical capability for one or more fluid components. Indeed, the emission source and detector could be configured to employ a Raman-based system for qualitative and quantitative analysis or a reflective-based system for qualitative analysis. While one preferred embodiment uses inexpensive LED's in a system based on transmission-absorption, the invention should not be so limited.

Other embodiments would have practical application. For example, a simple spectroscopic analyzer with an emission source and detector could be located in a structure remote from the pump. Such a device would be useful in the operating room, where drugs are given by IV push and only a qualitative analysis may be of interest. The term remote should be understood to mean that two structures are physically distinct. In terms of actual distance, they could be touching or they could be miles apart. Some examples will clarify this point. The emission source and detector could be located in a device adapted only for the spectroscopic analysis, and not for the rate monitoring function performed by the pump. Such a spectroscopic analyzer could be a separate housing clamped around the tubing of the IV administration set downstream from the infusion pump. An example would be somewhat like Figure 4 (or Fig. 8), except that the structure depicted in Figure 4 would be just the spectroscopic analyzer, and it would not have any infusion pump components or functions as described earlier but not show in the drawings. The analyzer could be mounted on the same stand as the pump or on a different stand, or, if light enough, simply clamped around the tubing with the intravenous fluid. It could be connected to the pump, where control and display of the spectroscopic analysis would still occur, or it could have its own control and display mechanisms. To quantitatively analyze the infusate, however, the analysis would require the infusion rate established by the pump. Some data link between the pump and the analyzer would be required. Like the embodiment in which the emission source and detector are mounted inside the pump, the electronic devices necessary to control and perform the spectroscopic analysis in a device separate from the infusion pump can be configured in a variety of ways. The analysis can be controlled and performed in the same device containing the emission source and detector, or various aspects of the control of the analysis and the analysis itself can be located remotely. The location and/or duplication of devices for control and data manipulation, such as keyboards, display panels, and hardware and software for spectroscopic analysis, are all a matter of choice. That choice is dictated by the needs of the medical services provider and the cost of any desired configuration. These choices do, however, permit the medical field to improve its ability to address all types of the medication errors that are discussed above.

Another example of variations to the invention can be seen in the tubing 154 and optical chamber 104. As noted earlier, one embodiment of the invention contemplates using just the tubing 154 with its cross-section determining the optical path length. Should one use the preferred embodiment in Figures 2 and 3, which include both tubing 154 and chamber 104, a variety of configurations are still possible. Chamber 104 need not be rectilinear in shape and need not have recessed windows 106. The primary consideration is maintaining optical path length A as fixed as possible in relation to the emission source and detector. Similarly, it is a matter of choice whether to fabricate chamber 104 and tubing 154 as a unitary structure from the same material. One possible embodiment could include an administration set that comes with two separate pieces of tubing that can be fixed to the optical chamber by medical personnel in a clinical setting. The chamber, in turn, can be placed inside the infusion pump by the medical personnel; it can be permanently constructed into the interior of the pump; or, it can remain outside the pump as part of a separate system for analyzing the fluid. Therefore, the term fluid path should be understood in its broadest sense to include those features necessary to give the term meaning. The fluid path may include a physical structure (or portion of it) containing the fluid or the tubing, it may represent a volume of space through which the fluid moves, or it may include something in between.

For simplicity, the invention has been described in terms a single fluid component. If more than one medication were provided intravenously to a patient, analysis of each medication would require a separate IV administration set with its own emission source and detector. This would be the case, for example, in intensive care units, where it is not unusual to provide a patient with four to six different medications, each associated with a separate infusion pump. It is, however, possible to develop a spectral database for a fluid with multiple components. A single emission source and detector could then be configured to provide the necessary wavelengths to simultaneously identify and quantify more than one pharmaceutical compound or fluid component being delivered through the IV administration set. In such a case, only one pump with one set of spectroscopic hardware and software would be necessary.

In yet other embodiments, the present invention could be adapted to functions other than the intravenous delivery of medication. One such function could involve kidney dialysis or similar procedures.

## Claims

1. An infusion pump system for delivering an intended intravenous fluid to a patient comprising:
a pump housing (70; 108) with an exterior and an interior;
a fluid path (54), at least a portion of which is disposed within the interior of the housing (70; 108) for conducting the intravenous fluid to be delivered;
means (80) for inputting the identification of at least one component of interest in the intended intravenous fluid;
a memory device (160) for storing at least one parametric value uniquely associated with said at least one component of interest.
a first emission source (146, 148, 150) positioned adjacent to said fluid path for directing electromagnetic radiation into the fluid to enable the electromagnetic radiation to interact with said at least one component of interest;
a first detector (112) positioned adjacent to the fluid path for detecting the electromagnetic radiation emerging from the fluid therein and for generating a signal for use in spectroscopically analyzing said at least one component of the fluid in the fluid path, and
means responsive to said signal for permitting said infusion only if said signal is substantially indicative of an intravenous fluid in the fluid path having said at least one component of interest.

2. The pump system of claim 1, further comprising at least one of electronic hardware and software adapted to process the detector signal to spectroscopically analyze the at least one component of interest of the fluid.

3. The pump system of claim 1, further comprising an optical chamber (104) disposed adjacent the emission source (146, 148, 150) and the detector (112) and through which the fluid passes during spectroscopic analysis.

4. The pump system of claim 3, wherein the optical chamber (104) is disposed between the emission source (146, 148, 150) and the detector (112).

5. The pump system of claim 2, wherein said inputting means (80) includes controls disposed on the exterior of the pump housing for controlling at least one parameter related to the spectroscopic analysis.

6. The pump system of claim 5, further comprising a display (78) disposed on the exterior of the pump housing for displaying the at least one parameter.

7. The pump system of claim 6, wherein one parameter is representative of the identity of the at least one component of interest of the fluid.

8. The pump system of claim 7, wherein the emission source (146, 148, 150) comprises at least two light-emitting diodes.

9. The pump system of claim 8, wherein a second parameter is representative of a maximum dosage of the at least one component of interest of the fluid.

10. The pump system of claim 9. further comprising a connection means (158) to at least one of remote electronic hardware and remote software adapted to spectroscopically analyze the at least one component of interest of the fluid.

11. The pump system of claim 3 wherein the optical chamber (104) is defined within a parallel-walled structure sized to fit within the interior of the pump housing (70; 108).

12. The pump system of claim 11 wherein the parallel-walled structure includes at least one optical window (106) through which the emission source (146, 148, 150) and detector (112) are optically coupled to the fluid.

13. The pump system of claim 11 wherein the parallel-walled structure includes a pair of opposing walls, with at least one optical window (106) being recessed into at least one of the walls.

14. The pump system of claim 11 wherein the optical chamber (104) is defined between at least two parallel walls, and includes an optical window (106) in each wall through which a respective one of the emission source (146, 148, 150) and detector (112) are optically coupled to the fluid.

15. The pump system of claim 11 wherein the pump housing (70; 108) includes a hinged door through which access to the parallel-walled structure is provided from the exterior of the housing.

16. The pump system of claim 15 wherein the hinged door includes means for substantially optically aligning the optical chamber (104) with the emission source (146. 148, 150) and detector (112) when the door is closed.

17. The pump system of claim 11 wherein the parallel-walled structure includes means for securing a fluid-conducting tube (54; 154) in substantial optical alignment with the emission source (146, 148. 150) and detector (112), and
means (46, 48, 52) for fluidically coupling said tube to a source (42) of intravenous fluid.

18. The pump system of claim 1 wherein the detector (112) includes means for detecting a spectral characteristic of fluid within the fluid path (54) at at least two substantially non-overlapping electromagnetic radiation frequency ranges, and for producing a signal indicative of the detected spectral characteristic associated with at least two of said frequency ranges.

19. The pump system of claim 18 including means for determining the ratio of the characteristic-indicative signals obtained for two substantially non-overlapping frequency ranges.

20. The pump system of claim 18 wherein the frequency ranges are respectively the frequency ranges within which the component of interest in the intended fluid produces a substantially minimum and substantially maximum signal level relative to all other frequency ranges.

21. The pump system of claim 1 wherein the inputting means is selected from the group consisting of at least one of (a) barcode reader means for scanning the barcode on a container of intravenous solution to transfer data indicative of the label-stated content of the container, (b) keypad entry means for entering data indicative of the desired content of the container, and (c) barcode reader means for scanning the barcode on a container of intravenous solution to transfer data indicative of the label-stated content of the container and keypad entry means for entering data indicative of the desired content of the container.

22. The pump system of claim 21 wherein the signal responsive means is responsive to the data supplied by the inputting means to permit infusion only if the inputted data and the spectral analysis of the fluid in the fluid path are all in accord.

23. The pump system of claim 1 including means for determining the concentration of the component of interest in the fluid contained in the fluid path.

24. The pump system of claim 23 including
means for inputting a parameter indicative of the desired concentration of the component of interest,
a second emission source for directing electromagnetic radiation at the fluid within the fluid path, and
a second detector for detecting the electromagnetic radiation emerging from the fluid in the fluid path, and
means for permitting delivery of fluid having said component of Interest to the patient only if the level of detected radiation is substantially equivalent to the level that would be detected from the desired concentration of the component of interest.

25. The pump system of claim 24 including memory means for storing digital values pertaining to the levels of detected radiation that are expected from fluids of respective concentrations of the component of interest, and
memory access means responsive to the parameter inputting means to provide the delivery-permitting means with the value of said desired concentration.

26. The pump system of claim 23 including means for inputting a parameter indicative of the desired concentration of the component of interest,
a second emission source for directing electromagnetic radiation at the fluid within the fluid path, and
a second detector for detecting the electromagnetic radiation emerging from the fluid in the fluid path,
integration means responsive to the infusion rate of the pump and the duration of the infusion to indicate the total dosage of the component of interest infused into the patient,
means for inputting the desired dosage of the component of interest, and
means responsive to the integration means and the inputting means for permitting delivery of the fluid to the patient.

27. The pump system of claim 24 wherein said first emission source is capable of emitting electromagnetic radiation over a first range of frequencies, and
wherein said first emission source functions as said second emission source by emitting electromagnetic radiation within a second frequency range that is substantially non-overlapping with said first frequency range.

28. The pump system of claim 27 wherein the first detector is capable of detecting electromagnetic radiation within said first range of frequencies, and
wherein said first detector functions as said second detector by detecting electromagnetic radiation within said second frequency range.

29. The pump system of claim 1 wherein the detector is disposed within the housing interior.

30. The pump system of claim 29 wherein the emission source is disposed within the housing interior.

31. The pump system of claim 1 wherein the emission source is disposed within the housing interior.

32. The pump system of claim 1, further comprising an optical chamber (104) through which the fluid passes during spectroscopic analysis, said optical chamber (104) being disposed adjacent the emission source (146, 148, 150) and the detector (112) and having at least one optical window (106) through which the fluid therein is optically coupled to the emission source and detector.

33. The pump system of claim 32 wherein the optical chamber defines a fixed length optical path between the emission source and detector.

34. The infusion pump system of claim 1, comprising:
an optical window (106) operatively connected to the system; and
a spectroscopic analyzer operatively connected to the system, the analyzer including an emission source (146, 148. 150) for directing electromagnetic radiation through the optical window at the fluid and a detector (112) for detecting the electromagnetic radiation emerging from the fluid.

35. The system of claim 34 wherein the intravenous fluid passes between the emission source and the detector.

36. The system of claim 35 wherein the emission source comprises at least two light-emitting diodes.

37. The system of claim 35 further comprising at least one of electronic hardware and software associated with the spectroscopic analyzer.

38. The system of claim 37 wherein the at least one of electronic hardware and software is remote from the spectroscopic analyzer.

39. An improved intravenous administration set for the infusion pump system of claim 1, wherein the fluid path comprises in at least a portion thereof tubing and including an optical window adapted to permit the transmittance and detection of electromagnetic radiation for use in a spectroscopic analysis of the fluid in operable relationship with at least a portion of the tubing.

40. The improved intravenous administration set of claim 39, further comprising an optical chamber containing the optical window.

## Patentansprüche

1. Infusionspumpsystem zur Freisetzung eines bestimmungsgemäßen intravenösen Fluids an einen Patienten, umfassend
ein Pumpengehäuse (70; 108) mit einem Inneren und einem Äußeren;
einem Fluidpfad (54), von dem mindestens ein Teil im Inneren des Gehäuses (70; 108) angeordnet ist, um das freizusetzende intravenöse Fluid zu leiten;
Mittel (80) zum Eingeben der Identifikation mindestens einer Komponente von Interesse im bestimmungsgemäßen intravenösen Fluid;
eine Speichervorrichtung (160) zum Speichern mindestens eines parametrischen Werts, der nur mit der mindestens einen Komponente von Interesse in Zusammenhang steht;
eine erste Emissionsquelle (146, 148, 150), angeordnet benachbart zu dem Fluidpfad zum Lenken elektromagnetischer Strahlung in das Fluid, um der elektromagnetischen Strahlung zu ermöglichen, mit der mindestens einen Komponente von Interesse wechselzuwirken;
einen ersten Detektor (112), angeordnet benachbart zum Fluidpfad, zum Detektieren der elektromagnetischen Strahlung, die darin vom Fluid auftritt, und zum Erzeugen eines Signals zur Verwendung zur spektroskopischen Analyse der mindestens einen Komponente des Fluids im Fluidpfad, und
Mittel, die auf das Signal reagieren, um nur dann Infusion zu ermöglichen, wenn das Signal im Wesentlichen ein intravenöses Fluid im Fluidpfad mit der mindestens einen Komponente von Interesse angibt.

2. Pumpsystem nach Anspruch 1, weiterhin umfassend mindestens eine elektronische Hardware oder Software, angepasst, um das Detektorsignal zu verarbeiten, um die mindestens eine Komponente von Interesse des Fluids spektroskopisch zu analysieren.

3. Pumpsystem nach Anspruch 1, weiterhin umfassend eine optische Kammer (104), angeordnet benachbart zur Emissionsquelle (146, 148, 150) und dem Detektor (112), und durch den das Fluid während der spektroskopischen Analyse passiert.

4. Pumpsystem nach Anspruch 3, worin die optische Kammer (104) zwischen der Emissionsquelle (146, 148, 150) und dem Detektor (112) angeordnet ist.

5. Pumpsystem nach Anspruch 2, worin das Eingabemittel (80) Kontrollen umfasst, die auf dem Äußeren des Pumpgehäuses angeordnet sind, um mindestens einen Parameter im Zusammenhang mit der spektroskopischen Analyse zu steuern.

6. Pumpsystem nach Anspruch 5, weiterhin umfassend ein Display (78), angeordnet auf dem Äußeren des Pumpgehäuses zum Anzeigen des mindestens einen Parameters.

7. Pumpsystem nach Anspruch 6, worin ein Parameter für die Identität der mindestens einen Komponente von Interesse des Fluids repräsentativ ist.

8. Pumpsystem nach Anspruch 7, worin die Emissionsquelle (146, 148, 150) mindestens zwei lichtemittierende Dioden aufweist.

9. Pumpsystem nach Anspruch 8, worin ein zweiter Parameter für die maximale Dosierung der mindestens einen Komponente von Interesse des Fluids repräsentativ ist.

10. Pumpsystem nach Anspruch 9, weiterhin umfassend ein Verbindungsmittel (158) zu mindestens einer der entfernten elektronischen Hardware oder entfernten Software, die angepasst ist, um die mindestens eine Komponente von Interesse des Fluids spektroskopisch zu analysieren.

11. Pumpsystem nach Anspruch 3, worin die optische Kammer (104) in einer Struktur mit parallelen Wänden definiert ist, die eine Größe aufweist, um in das Innere des Pumpgehäuses (70; 108) zu passen.

12. Pumpsystem nach Anspruch 11, worin die Struktur mit parallelen Wänden mindestens ein optisches Fenster (106) aufweist, durch das die Emissionsquelle (146, 148, 150) und der Detektor (112) optisch mit dem Fluid gekoppelt sind.

13. Pumpsystem nach Anspruch 11, worin die Struktur mit parallelen Wänden ein Paar entgegengesetzter Wände enthält, mit mindestens einem optischen Fenster (106), das in mindestens einer der Wände vertieft vorliegt.

14. Pumpsystem nach Anspruch 11, worin die optische Kammer (104) zwischen mindestens zwei parallelen Wänden definiert ist, und ein optisches Fenster (106) in jeder Wand umfasst, durch die jeweils eine der Emissionsquellen (146, 148, 150) und der Detektor (112) mit dem Fluid optisch gekoppelt sind.

15. Pumpsystem nach Anspruch 11, worin das Pumpengehäuse (70; 108) eine schwenkbare Tür enthält, durch die Zugang zur Struktur mit parallelen Wänden vom Äußeren des Gehäuses bereitgestellt ist.

16. Pumpsystem nach Anspruch 15, worin die schwenkbare Tür Mittel enthält, um die optische Kammer (104) mit der Emissionsquelle (146, 148, 150) und dem Detektor (112) im Wesentlichen optisch auszurichten, wenn die Tür geschlossen ist.

17. Pumpsystem nach Anspruch 11, worin die Struktur mit parallelen Wänden Mittel zum Sichern einer fluidleitenden Röhre (54; 154) in im Wesentlichen optischer Ausrichtung mit der Emissionsquelle (146, 148, 150) und dem Detektor (112) enthält, sowie
Mittel (46, 48, 52) zur Fluiditätskopplung der Röhre mit einer Quelle (42) des intravenösen Fluids.

18. Pumpsystem nach Anspruch 1, worin der Detektor (112) Mittel zum Detektieren einer spektralen Charakteristik des Fluids im Fluidpfad (54) mit mindestens zwei im Wesentlichen nicht überlappenden elektromagnetischen Strahlungsfrequenzbereichen enthält, und zur Erzeugung eines Signals, das die detektierte spektrale Charakteristik im Zusammenhang mit mindestens zwei der Frequenzbereiche angibt.

19. Pumpsystem nach Anspruch 18, umfassend Mittel zum Bestimmen des Verhältnisses der Charakteristik-angebenden Signale, erhalten aus zwei im Wesentlichen nicht-überlappenden Frequenzbereichen.

20. Pumpsystem nach Anspruch 18, worin die Frequenzbereiche jeweils Frequenzbereiche darstellen, in denen die Komponente von Interesse im bestimmungsgemäßen Fluid ein im Wesentlichen minimales und ein im wesentlichen maximales Signal-Niveau, bezogen auf sämtliche andere Frequenzbereiche, erzeugt.

21. Pumpsystem nach Anspruch 1, worin das Eingabemittel ausgewählt ist aus der Gruppe, bestehend aus mindestens einem von (a) einem Barcode-Lesemittel zum Einscannen des Barcodes auf einem Behälter mit intravenöser Flüssigkeit, um Daten zu transferieren, die den durch das Label angegebenen Inhalt des Behälters angeben, (b) Nummerblock-Eingabemittel zum Eingeben von Daten, die den gewünschten Inhalt des Behälters angeben, und (c) ein Barcode-Lesemittel zum Einscannen des Barcodes auf einem Behälter für intravenöse Lösung zum Transferieren von Daten, die den durch das Label angegeben Inhalt des Behälters angeben, sowie Nummernblock-Eingabemittel zum Eingeben von Daten, die den gewünschten Inhalt des Behälters angeben.

22. Pumpsystem nach Anspruch 21, worin das Signal-Reaktionsmittel auf Daten reagiert, die durch das Eingabemittel eingegeben werden, um nur dann Infusion zu ermöglichen, wenn die eingegebenen Daten und die spektrale Analyse des Fluids im Fluidpfad sämtlich in Übereinstimmung sind.

23. Pumpsystem nach Anspruch 1, enthaltend Mittel zum Bestimmen der Konzentration der Komponente von Interesse im Fluid, die im Fluidpfad enthalten ist.

24. Pumpsystem nach Anspruch 23, enthaltend
Mittel zum Eingeben eines Parameters, der die gewünschte Konzentration der Komponente von Interesse angibt,
eine zweite Emissionsquelle zum Lenken elektromagnetischer Strahlung auf das Fluid im Fluidpfad, und
einen zweiten Detektor zum Detektieren der elektromagnetischen Strahlung, die vom Fluid im Fluidpfad ausgeht, und
Mittel zum Ermöglichen des Freisetzens von Fluid mit der Komponente von Interesse an den Patienten, nur wenn das Niveau an detektierter Strahlung im Wesentlichen äquivalent zum Niveau ist, das von der gewünschten Konzentration der Komponente von Interesse detektiert werden würde.

25. Pumpsystem nach Anspruch 24, enthaltend Speichermittel zum Speichern digitaler Werte, betreffend die Niveaus detektierter Strahlung, die von den Fluids der jeweiligen Konzentrationen der Komponente von Interesse erwartet wird, und
Speicherzugangsmittel, die auf Parameter-Eingabemittel reagieren, um die Freisetzungszulassenden Mittel mit dem Wert der gewünschten Konzentration zu versorgen.

26. Pumpsystem nach Anspruch 23, enthaltend Mittel zur Eingabe eines Parameters, der die gewünschte Konzentration der Komponente von Interesse eingibt,
eine zweite Emissionsquelle zum Lenken elektromagnetischer Strahlung auf das Fluid im Fluidpfad, und
einen zweiten Detektor zum Detektieren der elektromagnetischen Strahlung, die vom Fluid im Fluidpfad ausgeht,
Integrationsmittel, die auf die Infusionsrate der Pumpe und die Dauer der Infusion reagieren, um die Gesamtdosierung der Komponente von Interesse, die dem Patienten durch Infusion verabreicht wird, anzuzeigen,
Mittel zum Eingeben der gewünschten Dosierung der Komponente von Interesse, und
Mittel, die auf die Integrationsmittel und die Eingabemittel zum Ermöglichen der Freigabe des Fluids in den Patienten reagieren.

27. Pumpsystem nach Anspruch 24, worin die erste Emissionsquelle dazu in der Lage ist, elektromagnetische Strahlung über einen ersten Bereich von Frequenzen zu emittieren, und
worin die erste Emissionsquelle als zweite Emissionsquelle fungiert, indem elektromagnetische Strahlung in einem zweiten Frequenzbereich emittiert wird, der im Wesentlichen nicht mit dem ersten Frequenzbereich überlappt.

28. Pumpsystem nach Anspruch 27, worin der erste Detektor die elektromagnetische Strahlung im ersten Bereich von Frequenzen detektieren kann, und
worin der erste Detektor als zweiter Detektor fungiert, indem elektromagnetische Strahlung im zweiten Frequenzbereich detektiert wird.

29. Pumpsystem nach Anspruch 1, worin der Detektor im Gehäuseinneren angeordnet ist.

30. Pumpsystem nach Anspruch 29, worin die Emissionsquelle im Gehäuseinneren angeordnet ist.

31. Pumpsystem nach Anspruch 1, worin die Emissionsquelle im Gehäuseinneren angeordnet ist.

32. Pumpsystem nach Anspruch 1, weiterhin umfassend eine optische Kammer (104), durch die das Fluid während der spektroskopischen Analyse passiert, wobei die optische Kammer (104) benachbart zur Emissionsquelle (146, 148, 150) und dem Detektor (112) angeordnet ist, und mindestens ein optisches Fenster (106) aufweist, durch das das Fluid darin optisch mit der Emissionsquelle und dem Detektor gekoppelt ist.

33. Pumpsystem nach Anspruch 32, worin die optische Kammer eine feste Länge des optischen Wegs zwischen der Emissionsquelle und dem Detektor definiert.

34. Infusionspumpsystem nach Anspruch 1, umfassend:
ein optisches Fenster (106), operativ verbunden mit dem System; und
einen spektroskopischen Analysator, operativ verbunden mit dem System, wobei der Analysator eine Emissionsquelle (146, 148, 150) zum Lenken elektromagnetischer Strahlung durch das optische Fenster auf das Fluid sowie einen Detektor (112) zum Detektieren der elektromagnetischen Strahlung, die vom Fluid ausgeht, enthält.

35. System nach Anspruch 34, worin das intravenöse Fluid zwischen der Emissionsquelle und dem Detektor passiert.

36. System nach Anspruch 35, worin die Emissionsquelle mindestens zwei lichtemittierende Dioden aufweist.

37. System nach Anspruch 35, weiterhin umfassend mindestens eine elektronische Hardware oder Software im Zusammenhang mit dem spektroskopischen Analysator.

38. System nach Anspruch 37, worin mindestens die elektronische Hardware oder Software vom spektroskopischen Analysator entfernt liegt.

39. Verbessertes intravenöses Verabreichungs-Set für das Infusionspumpsystem nach Anspruch 1, worin der Fluidpfad in mindestens einem Bereich hiervon Rohrleitungen aufweist sowie ein optisches Fenster enthält, das angepasst ist, um die Transmission und Detektion von elektromagnetischer Strahlung zur Verwendung in einer spektroskopischen Analyse des Fluids in einer Betriebsbeziehung mit mindestens einem Teil der Rohrleitung aufweist.

40. Verbessertes intravenöses Verabreichungs-Set nach Anspruch 39, weiterhin umfassend eine optische Kammer, enthaltend das optische Fenster.

## Revendications

1. Système de pompe à perfusion pour administrer un fluide intraveineux voulu à un patient, comprenant :
un logement de pompe (70; 108) comportant un extérieur et un intérieur ;
un trajet de fluide (54) dont une partie au moins est disposée à l'intérieur du logement (70; 108) pour guider le fluide intraveineux à administrer ;
des moyens (80) pour entrer l'identification d'au moins un composant d'intérêt contenu dans le fluide intraveineux voulu ;
un dispositif de mémoire (160) pour stocker au moins une valeur paramétrique associée de façon univoque audit composant d'intérêt,
une première source d'émission (146, 148, 150) positionnée de façon adjacente audit trajet de fluide pour diriger un rayonnement électromagnétique à l'intérieur du fluide afin de permettre au rayonnement électromagnétique d'interagir avec ledit composant d'intérêt ;
un premier détecteur (112) positionné de façon adjacente au trajet de fluide pour y détecter le rayonnement électromagnétique émergeant du fluide et pour générer un signal à utiliser dans l'analyse spectroscopique dudit composant du fluide contenu dans le trajet de fluide, et
des moyens sensibles audit signal pour autoriser ladite perfusion uniquement si ledit signal est sensiblement indicatif d'un fluide intraveineux dans le trajet de fluide comportant ledit composant d'intérêt.

2. Système de pompe selon la revendication 1, comprenant en outre un dispositif électronique matériel et/ou un dispositif électronique logiciel adapté pour traiter le signal de détecteur afin d'analyser par spectroscopie le composant d'intérêt du fluide.

3. Système de pompe selon la revendication 1, comprenant en outre une chambre optique (104) disposée de façon adjacente à la source d'émission (146, 148, 150) et au détecteur (112) et à travers laquelle passe le fluide pendant l'analyse spectroscopique.

4. Système de pompe selon la revendication 3, dans lequel la chambre optique (104) est disposée entre la source d'émission (146, 148, 150) et le détecteur (112).

5. Système de pompe selon la revendication 2, dans lequel lesdits moyens d'entrée (80) comprennent des commandes disposées sur l'extérieur du logement de pompe pour commander au moins un paramètre associé à l'analyse spectroscopique.

6. Système de pompe selon la revendication 5, comprenant en outre un dispositif d'affichage (78) disposé sur l'extérieur du logement de pompe pour afficher le paramètre.

7. Système de pompe selon la revendication 6, dans lequel un premier paramètre est représentatif de l'identité du composant d'intérêt du fluide.

8. Système de pompe selon la revendication 7, dans lequel la source d'émission (146, 148, 150) comprend au moins deux diodes électroluminescentes.

9. Système de pompe selon la revendication 8, dans lequel un second paramètre est représentatif d'un dosage maximal du composant d'intérêt du fluide.

10. Système de pompe selon la revendication 9, comprenant en outre des moyens de connexion (158) relié au dispositif électronique matériel et/ou au dispositif électronique logiciel adapté pour analyser par spectroscopie le composant d'intérêt du fluide.

11. Système de pompe selon la revendication 3, dans lequel la chambre optique (104) est définie à l'intérieur d'une structure à parois parallèles dimensionnée pour s'adapter à l'intérieur du logement de pompe (70 ; 108).

12. Système de pompe selon la revendication 11, dans lequel la structure à parois parallèles comprend au moins une fenêtre optique (106) à travers laquelle la source d'émission (146, 148, 150) et le détecteur (112) sont couplés optiquement au fluide.

13. Système de pompe selon la revendication 11, dans lequel la structure à parois parallèles comprend une paire de parois opposées, au moins une fenêtre optique (106) étant encastrée dans au moins l'une des parois.

14. Système de pompe selon la revendication 11, dans lequel la chambre optique (104) est définie entre au moins deux parois parallèles et comprend, dans chaque paroi, une fenêtre optique (106) à travers laquelle l'un ou l'autre de la source d'émission (146, 148, 150) et du détecteur (112) respectifs sont couplés optiquement au fluide.

15. Système de pompe selon la revendication 11, dans lequel le logement de pompe (70 ; 108) comprend une porte articulée permettant d'accéder à la structure à parois parallèles de l'extérieur du logement.

16. Système de pompe selon la revendication 15, dans lequel la porte articulée comprend des moyens pour aligner sensiblement optiquement la chambre optique (104) avec la source d'émission (146, 148, 150) et le détecteur (112) lorsque la porte est fermée.

17. Système de pompe selon la revendication 11, dans lequel la structure à parois parallèles comprend des moyens pour fixer un tube conducteur de fluide (54 ; 154) de façon sensiblement alignée optiquement avec la source d'émission (146, 148, 150) et le détecteur (112), et
des moyens (46, 48, 52) pour coupler de façon fluide ledit tube à une source (42) de fluide intraveineux.

18. Système de pompe selon la revendication 1, dans lequel le détecteur (112) comprend des moyens pour détecter une caractéristique spectrale de fluide à l'intérieur du trajet de fluide (54) dans au moins deux plages de fréquence de rayonnement magnétique sensiblement non chevauchantes, et pour produire un signal indicatif de la caractéristique spectrale détectée qui est associée à au moins deux desdites plages de fréquence.

19. Système de pompe selon la revendication 18, comprenant des moyens pour déterminer le rapport des signaux indicatifs de caractéristique, obtenus pour deux plages de fréquence sensiblement non chevauchantes.

20. Système de pompe selon la revendication 18, dans lequel les plages de fréquence sont respectivement les plages de fréquence à l'intérieur desquelles le composant d'intérêt contenu dans le fluide voulu produit un niveau de signal sensiblement minimal et sensiblement maximal relativement à toutes les autres plages de fréquence.

21. Système de pompe selon la revendication 1, dans lequel les moyens d'entrée sont choisis dans le groupe comprenant (a) des moyens de lecture à code à barres servant à balayer le code à barres inscrit sur un récipient de solution intraveineuse pour transférer des données indicatives du contenu indiqué sur l'étiquette du récipient, ou (b) des moyens d'entrée par clavier servant à entrer des données indicatives du contenu souhaité du récipient, ou bien (c) des moyens de lecture de code à barres servant à balayer le code à barres inscrit sur un récipient de solution intraveineuse pour transférer des données indicatives du contenu indiqué sur l'étiquette du récipient et des moyens d'entrée par clavier servant à entrer des données indicatives du contenu souhaité du récipient.

22. Système de pompe selon la revendication 21, dans lequel les moyens sensibles au signal sont sensibles aux données fournies par les moyens d'entrée pour autoriser la perfusion uniquement si les données entrées et l'analyse spectrale du fluide dans le trajet de fluide correspondent entièrement.

23. Système de pompe selon la revendication 1, comprenant des moyens pour déterminer la concentration du composant d'intérêt dans le fluide contenu dans le trajet de fluide.

24. Système de pompe selon la revendication 23, comprenant
des moyens pour entrer un paramètre indicatif de la concentration voulue du composant d'intérêt,
une seconde source d'émission pour diriger le rayonnement électromagnétique au niveau du fluide à l'intérieur du trajet de fluide, et
un second détecteur pour détecter le rayonnement électromagnétique émergeant du fluide dans le trajet de fluide, et
des moyens pour autoriser l'administration au patient de fluide comportant ledit composant d'intérêt uniquement si le niveau du rayonnement détecté est sensiblement équivalent au niveau qui serait détecté à partir de la concentration voulue du composant d'intérêt.

25. Système de pompe selon la revendication 24, comprenant des moyens de mémoire pour stocker des valeurs numériques se rapportant aux niveaux de rayonnement détecté escomptés de fluides de concentrations respectives du composant d'intérêt, et
des moyens d'accès à la mémoire sensibles aux moyens d'entrée de paramètre pour fournir aux moyens d'autorisation d'administration la valeur de ladite concentration voulue.

26. Système de pompe selon la revendication 23, comprenant des moyens pour entrer un paramètre indicatif de la concentration voulue du composant d'intérêt,
une seconde source d'émission pour diriger le rayonnement électromagnétique au niveau du fluide à l'intérieur du trajet de fluide, et
un second détecteur pour détecter le rayonnement électromagnétique émergeant du fluide dans le trajet de fluide,
des moyens d'intégration sensibles au débit de perfusion de la pompe et à la durée de la perfusion pour indiquer le dosage total du composant d'intérêt perfusé dans le patient,
des moyens pour entrer le dosage voulu du composant d'intérêt, et
des moyens sensibles aux moyens d'intégration et aux moyens d'entrée pour autoriser l'administration du fluide au patient.

27. Système de pompe selon la revendication 24, dans lequel ladite première source d'émission est capable d'émettre un rayonnement électromagnétique sur une première plage de fréquences, et
dans lequel ladite première source d'émission fonctionne à la manière de ladite seconde source d'émission en émettant un rayonnement électromagnétique dans une seconde plage de fréquences qui ne chevauche sensiblement pas ladite première plage de fréquences.

28. Système de pompe selon la revendication 27, dans lequel le premier détecteur est capable de détecter rayonnement électromagnétique dans ladite première plage de fréquences, et
dans lequel ledit premier détecteur fonctionne à la manière dudit second détecteur en détectant le rayonnement électromagnétique à l'intérieur de ladite seconde plage de fréquences.

29. Système de pompe selon la revendication 1, dans lequel le détecteur est disposé dans l'intérieur du logement.

30. Système de pompe selon la revendication 29, dans lequel la source d'émission est disposée dans l'intérieur du logement.

31. Système de pompe selon la revendication 1, dans lequel la source d'émission est disposée dans l'intérieur du logement.

32. Système de pompe selon la revendication 1, comprenant en outre une chambre optique (104) à travers laquelle passe le fluide pendant une analyse spectroscopique, ladite chambre optique (104) étant disposée de façon adjacente à la source d'émission (146, 148, 150) et au détecteur (112) et comportant au moins une fenêtre optique (106) à travers laquelle le fluide qui y est contenu est couplé optiquement à la source d'émission et au détecteur.

33. Système de pompe selon la revendication 32, dans lequel la chambre optique définit un trajet optique de longueur fixe entre la source d'émission et le détecteur.

34. Système de pompe à perfusion selon la revendication 1, comprenant :
une fenêtre optique (106) reliée fonctionnellement au système ; et
un dispositif d'analyse spectroscopique relié fonctionnellement au système, le dispositif d'analyse comprenant une source d'émission (146, 148, 150) pour diriger un rayonnement magnétique à travers la fenêtre optique au niveau du fluide et un détecteur (112) pour détecter le rayonnement électromagnétique émergeant du fluide.

35. Système selon la revendication 34, dans lequel le fluide intraveineux passe entre la source d'émission et le détecteur.

36. Système selon la revendication 35, dans lequel la source d'émission comprend au moins deux diodes électroluminescentes.

37. Système selon la revendication 35, comprenant en outre un dispositif électronique matériel et/ou un dispositif électronique logiciel associé(s) au dispositif d'analyse spectroscopique.

38. Système selon la revendication 37, dans lequel le dispositif électronique matériel et/ou le dispositif électronique logiciel se situe(nt) à distance du dispositif d'analyse spectroscopique.

39. Nécessaire à perfusion intraveineuse amélioré pour le système de pompe à perfusion selon la revendication 1, dans lequel le trajet de fluide comprend, dans au moins une de ses parties, une tubulure et comprenant une fenêtre optique adaptée pour permettre la transmittance et la détection d'un rayonnement électromagnétique destiné à être utilisé dans une analyse spectroscopique du fluide, en relation fonctionnelle avec au moins une partie de la tubulure.

40. Nécessaire à perfusion intraveineuse amélioré selon la revendication 39, comprenant en outre une chambre optique contenant la fenêtre optique.
